Europäisches Patentamt

European Patent Office

Office européen des brevets

(11) Publication number: **0 390 388**
**A2**

# EUROPEAN PATENT APPLICATION

(21) Application number: **90302913.0**

(22) Date of filing: **19.03.90**

(51) Int. Cl.5: **A61M 5/145**

(30) Priority: **28.03.89 GB 8906904**

(43) Date of publication of application:
**03.10.90 Bulletin 90/40**

(84) Designated Contracting States:
**BE DE FR GB IT NL SE**

(71) Applicant: **The BOC Group plc**
**Chertsey Road**
**Windlesham Surrey GU20 6HJ(GB)**

(72) Inventor: **Earle, Stephen Richard**
**134 Peckover Drive**
**Pudsey, West Yorkshire LS28 8EG(GB)**
Inventor: **Gray, John Martyn**
**7 Broadlands, Shann Park**
**Keighley, West Yorkshire BD20 6HX(GB)**
Inventor: **Burns, Paul Stephen**
**150 Pasture Lane**
**Clayton, Bradford BD14 6NQ(GB)**

(74) Representative: **Gough, Peter et al**
**c/o THE BOC GROUP PLC Patent Department**
**Chertsey Road**
**Windlesham Surrey GU20 6HJ(GB)**

(54) Improvements in infusion pumps.

(57) An infusion pump includes a system for sensing the near end and end of syringe positions of a syringe 10 piston 6 in a syringe 12 by means of a permanent magnet 14 located in a disengagement head 8. The magnet 14 as it passes a switch 16 located adjacent the syringe 12 within the pump case, closes the switch 16 thereby transmitting a signal to a microprocessor 20. The signal when received by the microprocessor 20 causes the microprocessor 20 to store pulses coming from the infusion pump motor encoder 22. The sum of the pulses received from the encoder 22 is compared with a preset value which once reached causes an alarm to be operated thereby indicating a near end or end of syringe position of the syringe piston 6 relative to the syringe 12.

EP 0 390 388 A2

K

The present invention relates to fluid delivery apparatus and in particular to apparatus for the infusion of medical liquids into patients.

Infusion pumps operate by driving a syringe plunger along the interior of the syringe thereby expelling medical liquids contained therein and into the body of a patient at a predetermined rate. It is known for infusion pumps to include a system for sensing two positions of a plunger namely "a near end position" and "an end of syringe position". Such sensing systems can be complicated and expensive particularly when the infusion pump is designed to work with different sizes and makes of syringe.

It is an aim of the present invention to provide an infusion pump with a system for sensing different relative positions of a syringe plunger in order to take into account difference clinical requirements, syringe sizes and makes with a minimum of hardware.

According to the present invention, an infusion pump comprises a motor coupled to means for engaging and driving a plunger forming part of a syringe when said syringe is operatively loaded in the infusion pump, and is characterised by the means carrying therewith means for operating a switch located adjacent the syringe, said switch, when operated, transmitting a signal to a microprocessor to store the number of pulses from the motor and compare the sum of said pulses against a predetermined pulse count, such that, when the sum of the pulses reaches the predetermined pulse count either an alarm is generated and/or the motor is stopped.

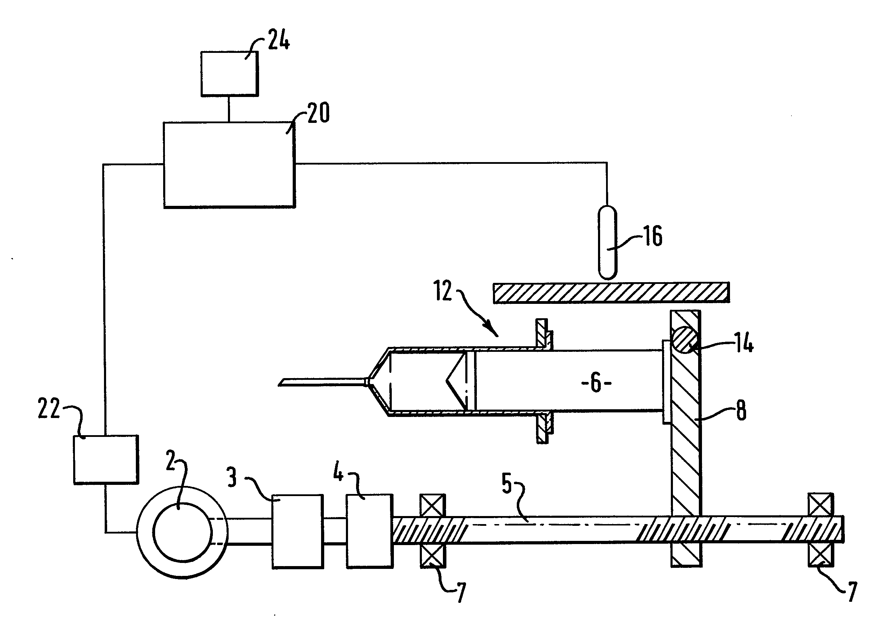

An embodiment of the invention will now be described, by way of example, reference being made to the Figure of the accompanying diagrammatic drawing which is a sketch of an infusion pump.

As shown, an infusion pump includes a motor 2 connected through a gearbox 3 and a coupling 4 to a drive screw 5 mounted for rotation in bearings 7. A disengagement head 8 is mounted for longitudinal movement along drive screw 5 and at its free end engages an end of a piston 6 forming part of a syringe 12.

The disengagement head 8 has mounted thereon for movement therewith a permanent magnet 14. Located adjacent the syringe 12 inside the pump case is a reed switch 16. The reed switch 16 is electrically connected to a microprocessor 20 which is also electrically connected to an encoder 22 incorporated in the motor 2.

In use, when it is desired to expel medical liquid in the syringe 12 and deliver said liquid to a patient the disengagement head 8 is moved to engage the end of the piston 6, the motor 2 is operated thereby driving piston 6 to the left (as shown) into the syringe 12. The disengagement head 8 will cause the piston 6 to expel fluid in the syringe 12 from the left hand end (as shown) thereof at a predetermined rate in a manner known per se. The piston 6 will travel along the interior of the syringe 12 and the permanent magnet 14 mounted on the disengagement head 8 will eventually, as it passes the reed switch 16, close said reed switch (the trigger point).

The closure of the reed switch 16 generates a signal to the microprocessor 20 causing the microprocessor 20 to begin storing the number of pulses transmitted from the motor encoder 22 into a counter 24. From the position at which the permanent magnet 14 closes the reed switch 16, that is the trigger position, the number of pulses to the required near end and end of syringe position is calculated by the pump manufacturer and stored in the microprocessor's memory. These calculated numbers are then continuously compared with the value in the counter 24 once the reed switch 16 is closed until they are the same. At this point an alarm is actuated for near end or end of syringe position depending on the count status.

In the above described embodiment the alarm system described is accurate in the measurement of the distance from the trigger point, that is, where the magnet 14 closes the reed switch 16, of better than plus or minus 0.001mm due to the required resolution of any pump speed control system. The only weakness of the system is the actual size of the syringe 12 being used with regard to the repeatability of the alarm position between syringes.

The above described alarm system prevents the pump being turned on and started whilst the reed switch 16 is closed up to the end of the disengagement head stroke, since unless the pump can guarantee the position of the disengagement head 8 when the reed switch 16 closes and the pulse count starts the end position of the syringe plunger cannot be controlled relative to its body.

**Claims**

1. An infusion pump comprising a motor 2 coupled to means 5, 8 for engaging and driving a plunger 6 forming part of a syringe 12 when said syringe 12 is operatively loaded in the infusion pump, characterised by the means 8 carrying therewith means 14 for operating a switch 16 located adjacent the syringe 12, said switch 16, when operated, generating a signal which is transmitted to a microprocessor 20 which causes the microprocessor 20 to store the number of pulses from the motor 2 and compare the sum of said pulses against a predetermined pulse count, such that, when the sum of the pulses reaches said predetermined pulse count either an alarm is given and/or the motor 2 is stopped.

2. An infusion pump as claimed in claim 1, characterised in that the switch 16 is a reed switch and the operating means is a magnet 14 carried for movement with a disengagement head 8 forming part of the engaging and driving means 5, 8.

EP 0 390 388 A2